# FASCICULE DE BREVET EUROPEEN

(11) **EP 0 587 473 B1**
(45) Date de publication et mention de la délivrance du brevet: **11.11.1998**
(21) Numéro de dépôt: 93402095.9
(22) Date de dépôt: 25.08.1993
(51) Int. Cl.: C07D 471/04, A61K 31/435, C07D 471/14, C07D 495/04, C07D 495/14

(54) **Dérivés condensés de la pyridine comme inhibiteurs de l'effet des radicaux libres**
Kondensierte Pyridin Derivate als Hemmer von Effekten freier Radikaler
Condensed pyridine derivatives as inhibitors of the effects of free radicals

(30) Priorité: 27.08.1992 FR 9210329
(43) Date de publication de la demande: 16.03.1994
(73) Titulaire: SANOFI, 75008 Paris (FR)
(72) Inventeur: Bachy, André, F-31100 Toulouse (FR); Fraisse, Laurent, F-64110 Jurancon (FR); Keane, Peter, F-31120 Portet sur Garonne (FR); Mendes, Etienne, F-31000 Toulouse (FR); Vernieres, Jean-Claude, F-31600 Muret (FR); Simiand, Jacques, F-31600 Muret (FR)
(74) Mandataire: Le Guen, Gérard

(56) Documents cités:
- FR-A- 2 246 271
- CHEMICAL ABSTRACTS, vol. 108, no. 3, 1988, Columbus, Ohio, US; abstract no. 21749q, J. SHARADA ET AL. 'Synthesis and biological activity of furoquinolines: 2-a royl-4-methyl/4,6-dimethyl-3-phenylfuro(3, 2-c)quinolines.' page 587 ; & INDIAN J. PHARM. SCI. vol. 49, no. 1 , 1987 pages 17 - 21

## Description

La présente invention concerne des dérivés d'acides pyrrolo[3,2-c] pyridine 2-carboxylique condensés avec un hétérocycle aromatique et leurs analogues soufrés, leurs procédés de préparation et les compositions pharmaceutiques qui les comprennent comme principes actifs.

Ces nouveaux composés sont, notamment, des inhibiteurs de l'effet des radicaux libres oxygénés biologiques, comme l'anion superoxyde O₂^{.-} ou le radical hydroxyle ; on sait que lorsque ces radicaux libres, nécessairement présents chez l'homme et l'animal, se forment en quantité trop importante pour qu'ils soient éliminés rapidement par les mécanismes physiologiques habituels, ils attaquent et détruisent les constituants des cellules, comme les acides nucléiques, les protéines, ou les membranes. Les tissus touchés sont très divers et de nombreux auteurs considèrent que les radicaux libres sont à l'origine de multiples maladies dégénératives allant des maladies inflammatoires ou auto-immunes à l'insuffisance respiratoire, aux ischémies cardiaque, cérébrale ou intestinale ; ils sont aussi impliqués dans les phénomènes dégénératifs du vieillissement.

FR 2 246 271 concerne des acides pyrazolo[3,4-b]thiéno[2,3-d]pyridine-2-carboxyliques et leurs esters comme anti-inflammatoires. Ce document ne fait nullement allusion à l'action éventuelle de ces composés sur les radicaux libres oxygénés.

Indian.J. Pharm. Sci (1987), 49(1), 17-21 décrit la synthèse d'aroylfuroquinoléines qui sont distinctes des composés revendiqués. L'activité biologique de ces dérivés de quinoléines n'est pas mentionnée.

Les composés de l'invention répondent à la formule I : 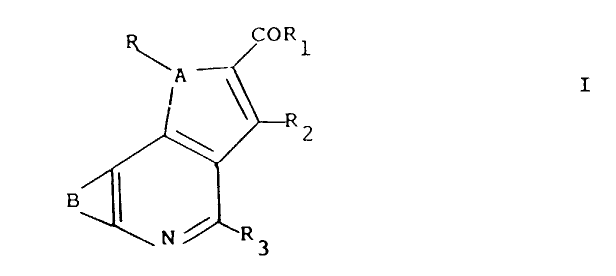 dans laquelle
R₁ représente OH, (C₁-C₁₂)alkyle, (C₁-C₁₂)alkoxy, benzyloxy, phényle, benzyle, (C₁ -C₄)alkylNZ₁Z₂ ou NZ₁Z₂ ;
R₂ représente OH, SH, (C₁-C₄)alkoxy, (C₁-C₄)alkylthio ou NZ₁Z₂ ;
R₃ représente H, (C₁-C₄)alkyle, (C₁-C₄)alkylthio, (C₁-C₄)alkoxy, phényle ou benzyle ;
A représente S, et dans ce cas R ne représente rien, ou A représente N et R représente H ou (C₁-C₄)alkyle qui peut être substitué par phényle ou NZ₁Z₂ ;
B représente un noyau aromatique condensé avec le noyau pyridyle, et peut être phényle, pyridyle ou thiényle pour former respectivement un groupe tricyclique pyrrolo [3,2-c]quinoléine, pyrrolo[3,2-c]naphtyridine ou pyrrolo[3,2-c]thiénopyridine lorsque A représente N, ou thiéno[3,2-c]quinoléine, thiéno[3,2-c]naphtyridine ou thiéno[3,2-c]thiénopyridine lorsque A représente S ;
B est éventuellement substitué par un ou des groupes choisis parmi halogéno, (C₁-C₆)alkyle, (C₁-C₆)alkoxy, CF₃, CH₂NZ₁Z₂ ou NZ₁Z₂ ; et
Z₁ et Z₂ représentent, indépendamment les uns des autres, H, (C₁-C₆)alkyle, formyle, benzyle, ou ils forment avec l'atome d'azote auquel ils sont attachés un hétérocycle saturé, éventuellement interrompu par un hétéroatome choisi parmi O, S ou N, ce dernier pouvant être substitué par (C₁-C₈)alkyle, benzyle, phényle, ou diphénylméthyle, étant entendu que lorsque A est S, R ne représente rien, R₁ est -OCH₃, R₃ est H et B est thiényle, R₂ ne peut pas représenter -OH ou -OCH₃;
ainsi que les sels de ces composés avec un acide ou une base.

Les groupes alkyle et alkoxy peuvent être linéaires ou ramifiés ou former un cycle, notamment cyclopentyle ou cyclohexyle.

Comme halogène, on préfère F ou Cl.

Lorsque le groupe NZ₁Z₂ représente un hétérocycle saturé, il peut être un pyrrolidinyle, piperidinyle, morpholino, hexahydroazépino, piperazino, ou piperazino substitué en position 4 par (C₁-C₈)alkyle, benzyle, phényle, ou diphénylméthyle.

Parmi les composés de l'invention, on préfère ceux de formule II : 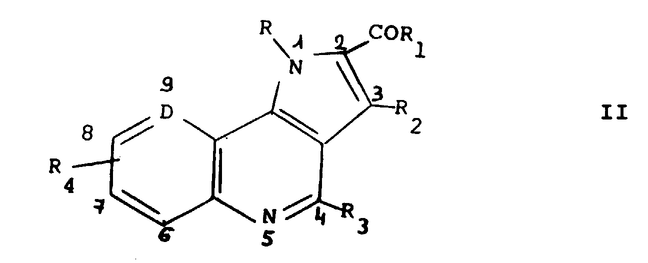 dans laquelle R, R₁, R₂, R₃ sont comme dans la formule I et D représente N ou mieux C, tandis que R₄ représente NZ₁Z₂, et plus particulièrement ceux dans lesquels R₄ est en position 8, en ortho de D et, parmi ceux-ci, de préférence, les composés dans lesquels R₂ est NZ'₁Z'₂, Z'₁ et Z'₂ représentant, indépendamment l'un de l'autre, H ou (C₁-C₆)alkyle. Parmi ces derniers composés, ceux pour lesquels COR₁ est en plus un groupe ester, sont préférés.

Un autre groupe de composés préférés est constitué des composés de formule II dans laquelle R, R₁, R₂, R₃ sont comme dans la formule I et D représente N, tandis que R₄ représente NZ₁Z₂, Z₁ et Z₂ représentant indépendamment l'un de l'autre H, (C₁-C₆)alkyle ou bien Z₁ et Z₂ formant avec l'atome d'azote auquel ils sont rattachés un groupe pipérazino qui peut être substitué sur l'azote par (C₁-C₈)alkyle, phényle ou diphénylméthyle.

Les composés de formule I peuvent être préparés en utilisant des procédés dont les principes sont connus en eux-mêmes, à partir de quinoléines, naphtyridines ou thiénopyridines convenablement substituées.

Selon un procédé on cyclise, dans une réaction de Dieckmann, le composé de formule 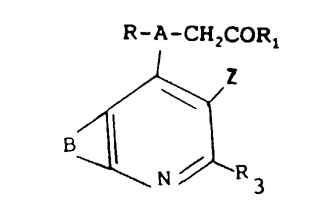 dans laquelle A, B, R, R₁ et R₃ ont la même signification que dans la formule I, et Z représente un groupe alkoxycarbonyle pour former un composé de formule I dans laquelle R₂ représente OH, ou Z représente un groupe cyano pour former un composé de formule I dans laquelle R₂ représente NH₂.

Selon un autre procédé on chauffe, en présence de phénol, les composés de formules 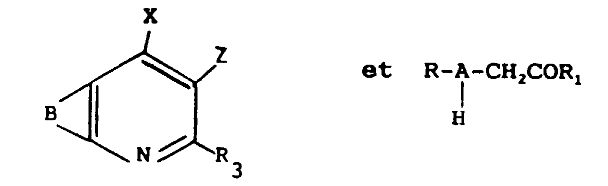 dans lesquelles A, B, R, R₁ et R₃ ont la même signification que dans la formule I, X représente Cl et Z représente un groupe alkoxycarbonyle pour former un composé de formule I dans laquelle R₂ représente OH, ou Z représente un groupe cyano pour former un composé de formule I dans laquelle R₂ représente NH₂.

Pour obtenir les dérivés pyrroliques de formule I dans lesquels A est N et R₂ est OH, on fait réagir une amine de formule III :

RNHCH₂COR₁ III

dans laquelle R et R₁ ont la même signification que dans la formule I, à l'exclusion de R₁ = OH, avec le composé de formule IV : 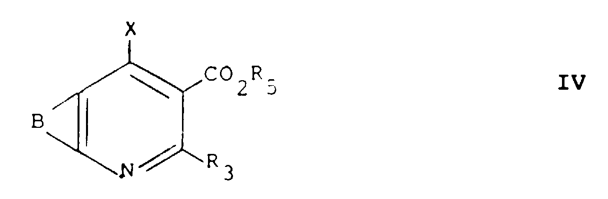 dans laquelle X représente Cl ou Br, R₅ représente (C₁-C₃)alkyle et B, R₃ ont la même signification que dans la formule I, pour obtenir le composé de formule V : 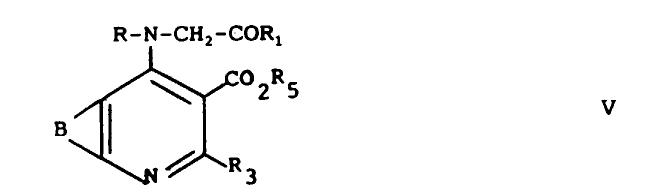 dans laquelle R, R₁, R₃, R₅ et B sont comme dans les formules III et IV, et que l'on cyclise dans une réaction de Dieckmann par action d'une base organique ou minérale, telle que (C₂H₅)₃N, C₂H₅ONa, t-C₄H₉ONa, NaH ou KOH dans un solvant non aqueux, tel qu'un alcool, un éther comme le dioxanne, ou le diméthylformamide, de préférence à une température comprise entre 0°C et 80°C.

Pour obtenir les dérivés pyrroliques de formule I dans lesquels A est N et R₂ est NH₂, on fait réagir une amine de formule III sur un composé de formule VI : 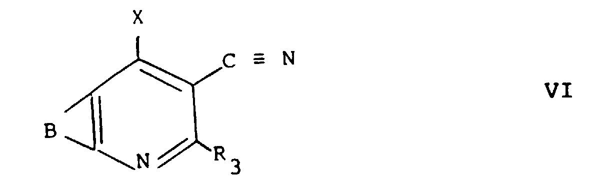 dans laquelle X est Cl ou Br, et R₃ et B ont la même signification que dans la formule I, pour obtenir le composé de formule VII : 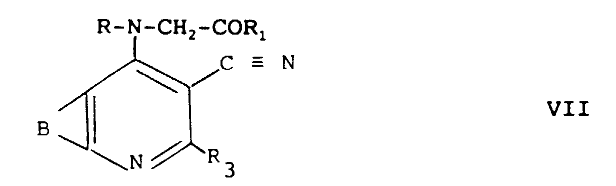 dans laquelle R, R₁, R₃ et B sont comme dans la formule I, et qui est cyclisé par une réaction de Dieckmann.

On peut aussi obtenir directement les composés de formule I par réaction des pyridines condensées de formule IV ou VI et des amines de formule III en présence d'un équivalent de phénol et d'un équivalent molaire d'une base organique ou minérale, en général sans solvant, à une température supérieure à 100°C environ.

Lorsque, dans le composé de formule I que l'on veut obtenir, R est différent de H, on peut substituer l'azote du pyrrole du composé de formule I dans lequel R = H, par action d'un halogénure RX en présence d'une base forte, telle que NaH, dans un solvant aprotique polaire ou dans un milieu biphasique en présence d'un catalyseur de transfert de phase.

Pour obtenir les composés thiophéniques de formule I dans lesquels A représente S, on fait réagir sur le composé de formule IV ou sur celui de formule VI un dérivé de formule HS-CH₂-COR₁ (à la place de l'amine RNHCH₂-COR₁ de formule III).

Pour obtenir les composés de formule I dans lesquels R₁ est OH, on hydrolyse, en milieu acide aqueux de préférence, les esters correspondants dans lesquels R₁ est alkoxy, par exemple tertiobutoxy, tandis que pour obtenir les composés dans lesquels R₁ est NZ₁Z₂, on peut faire réagir l'amine HNZ₁Z₂ sur l'ester correspondant dans les conditions habituelles d'obtention des amides secondaires ou tertiaires ; on préfère cependant faire réagir directement l'amide de formule III : RNHCH₂-CONZ₁Z₂ sur le composé de formule IV.

Les composés de formule I dans lesquels R₂ représente alkoxy sont préparés à partir de ceux dans lesquels R₂ est OH par réaction avec un halogénure d'alkyle convenable en présence d'une base forte telle que NaH ou C₂H₅ONa dans les conditions habituelles de formation d'un éther oxyde, éventuellement après protection de l'azote du pyrrole.

Les composés de formule I dans lesquels R₂ représente NZ₁Z₂ avec Z₁ et Z₂ différents de H, sont préparés par les méthodes connues de substitution des amines primaires aliphatiques.

Les pyridines condensées de formules IV ou VI sont préparées à partir des amines BNH₂ par des méthodes classiques décrites notamment dans J. Med.Chem. 22, 816-823 (1979) par l'intermédiaire des dérivés hydroxylés correspondants de formules IV ou VI dans lesquelles X représente OH, qui donnent, par exemple, le composé dans lequel X = Cl par action de POCl₃.

Les composés de formule VIII : 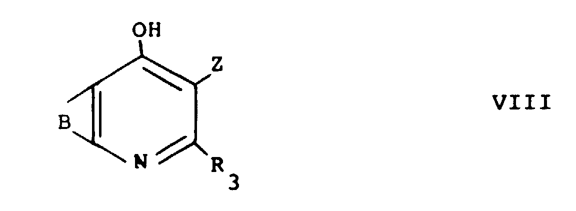 dans laquelle Z représente un groupe éthoxycarbonyle ou cyano peuvent être préparés selon le schéma réactionnel suivant :

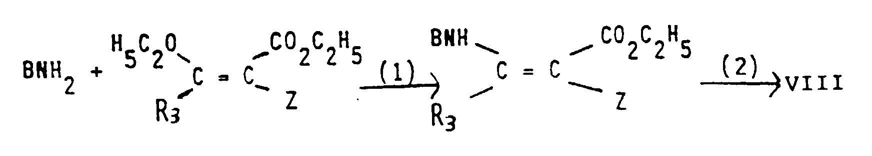

La première étape est réalisée en général sans solvant tandis que la cyclisation est effectuée dans un solvant de très haut point d'ébullition, le Dowtherm® ou l'oxyde de diphényle.

Certains des dérivés de formules V et VII qui en dérivent ont été décrits dans EP-A-0 205 362 et EP-A-0 346 208. Les autres peuvent être préparés par des modes opératoires analogues. Un procédé de préparation d'aminocétones de formule III est décrit notamment dans Tetrahedron Letters 25 2977-2980 (1984) ; les aminoesters sont des composés bien connus.

Les compositions pharmaceutiques qui contiennent comme principes actifs au moins un composé de formule I ou l'un de leurs sels avec un acide pharmaceutiquement acceptable sont un autre objet de l'invention. Ces compositions peuvent contenir les excipients classiquement utilisés pour obtenir des formes pharmaceutiques administrables par voie orale, rectale ou par injection intraveineuse ou intramusculaire.

Pour l'administration par voie orale, sous forme de comprimés, gélules ou granules ou encore de sirops,la dose unitaire sera comprise entre 5 et 200 mg, selon l'activité du composé, la nature de la maladie à traiter et l'état du patient.

Pour l'administration par voie parentérale, on utilisera de préférence des sels hydrosolubles des composés de formule I, ou à défaut, on introduira dans la composition des produits connus comme adjuvants de solubilisation ; la dose unitaire sera alors comprise entre 1 mg et 30 mg pour un volume de 1 à 5 cm³.

Les compositions pharmaceutiques selon l'invention seront administrées à titre préventif ou curatif chez l'homme ou l'animal pour diminuer ou supprimer les conséquences toxiques de la présence de radicaux libres oxygénés et traiter les troubles qui en résultent. Ceux-ci concernent des organes variés, et les compositions de l'invention pourront être utilisées dans le traitement des maladies rhumatismales comme l'arthrose, des maladies pulmonaires, de maladies cardio-vasculaires comme l'artériosclérose ou des conséquences d'ischémies, des troubles oculaires comme la cataracte, ou encore de déficits neurologiques, dont la maladie d'Alzheimer. Pour avoir connaissance des nombreuses utilisations thérapeutiques potentielles de ces composés, on pourra se référer aux ouvrages et articles publiés récemment, notamment à : "Free radicals in molecular biology, aging and disease", publié par Raven Press (New York), ou à "CRC handbook of free radicals and antioxidants in biomedecine", publié par CRC Press (Boca Raton, Florida, U.S.A.).

Néanmoins, les applications thérapeutiques des composés selon l'invention ne sauraient être limitées aux indications mentionnées ; les composés pourront être utilisés à titre préventif ou curatif dans toutes les situations où un excès de radicaux libres oxygénés peut perturber l'état physiologique normal.

L'activité de composés selon l'invention sur les radicaux libres oxygénés biologiques a été mise en évidence in vitro et in vivo.

In vitro, on a étudié leur action sur la formation du dialdéhyde malonique au cours de la décomposition des endopéroxydes résultant de l'oxydation, en présence de sel de fer, des acides gras polyinsaturés contenus dans les phospholipides des membranes cellulaires. On a utilisé une méthode classique, décrite notamment dans CRC Handbook of Methods for Oxygen Radical Research, p. 203-207 (1985), CRC Press-Boca Raton (Florida, U.S.A.).

In vivo, on a montré que ces composés s'opposaient aux effets toxiques du cyanure de potassium chez la souris et diminuaient l'hyperglycémie due à l'administration d'alloxane chez la souris.

En effet, on admet que lors d'une intoxication par le KCN, les désordres cellulaires et les troubles nerveux sont la conséquence d'une augmentation du calcium intracellulaire, mais aussi de l'oxydation des membranes lipidiques des cellules cérébrales et notamment dans J. Med. Chem. 33 1145-1151 (1990) et Arzneim. Forsch., Drug Research 39 445-450 (1989), l'antagonisme de la toxicité du KCN a été utilisé pour prédire une activité antioxydante dans le cerveau.

Par ailleurs, on sait que l'administration d'alloxane à l'animal entraîne une hyperglycémie due à la destruction des cellules bêta du pancréas par les radicaux oxygénés, dont la formation est déclenchée par la présence d'alloxane, et il a été montré que divers capteurs des radicaux libres oxygénés s'opposent à cette hyperglycémie, notamment dans Biochem. Pharmacol. 34, 3601-3603 (1985) et Fundamental Clinical Pharmacology 3, 323-328 (1989).

Dans le test au dialdéhyde malonique, la concentration inhibitrice à 50% (CI₅₀) est, pour les composés de l'invention, généralement comprise entre 0,1 µM et 10 µM. Dans le test au cyanure de potassium, la dose efficace 50 (DE₅₀) est comprise entre 2 mg/kg et 30 mg/kg environ par voie intraveineuse chez la souris, tandis que dans le test à l'alloxane, elle est comprise entre 0,1 mg/kg et 10 mg/kg par voie intraveineuse chez la souris.

Dans ce qui suit, on décrit des exemples de l'invention, avec pour certains, leur méthode de préparation détaillée.

### EXEMPLE 1

### 3-hydroxy -1 H - pyrrolo[3,2-c]quinoléine-2 carboxylate d'éthyle (A = N ; B = C₆H₄ ; R = H ; R₁ = OC₂H₅ ; R₂ = OH ; R₃ = H)

6 g de [4-(3-éthoxycarbonyl)quinolyl] 2-aminoacétate d'éthyle (F = 114°C) en solution dans 80 ml de toluène sec sont ajoutés en 30 minutes à 2,5 g de tert-butylate de potassium dans 80 ml de tert-butanol anhydre.

Le mélange est agité pendant 10 heures sous azote puis versé dans 200 ml d'eau glacée. On introduit dans la phase aqueuse basique une solution aqueuse de HCl N jusqu'à pH8. Le précipité apparu est filtré, lavé à l'éthanol absolu puis recristallisé dans le mélange diméthylformamide/éthanol (50/50). Les cristaux sont séchés à 130°C sous vide. Rendement 30% - F>250°C.

### EXEMPLE 2

### 8-chloro-2-(N,N-dipropylcarbamoyl)3-hydroxy 1-méthyl pyrrolo[3,2-c]quinoléine (A = N; B = 8-ClC₆H₃; R = CH₃ ; R₁ = N(C₃H₇)₂; R₂ = OH, R₃ = H)

A 1,94 g de 6-chloro 4-(N-(N,N dipropylcarbamoylméthyl) N-méthyl-amino) quinoléine-3 carboxylate d'éthyle en solution dans 30 ml de tétrahydrofuranne, on ajoute peu à peu 0,66 g de tert-butylate de potassium ; le mélange est agité pendant 2 heures, puis on concentre l'ensemble à sec. Le précipité est solubilisé dans l'eau ; la phase aqueuse est acidifiée par addition d'une solution aqueuse de HCl N jusqu'à pH7. Le produit attendu précipite ; il est purifié par recristallisation dans l'acétate d'éthyle ; F = 180°C (Rendement 58%).

### EXEMPLE 3

### 8-(N,N-diéthylamino) 2-(N,N-dipropylcarbamoyl)3-hydroxy moyl)3-hydroxy 1-méthyl pyrrolo[3,2-c]quinoléine (A = N; B = 8-(C₂H₅)₂NC₆H₃; R = CH₃; R₁ = N(C₃H₇)₂ ; R₂ = OH);

Dans une solution de 20 ml de tert-butanol et de 1,12 g de tert-butylate de potassium, on additionne sous azote 4,42 g de 6-(N,N-diéthyl amino) 4-(N-(N,N dipropylcarbamoylméthyl) N-méthyl-amino) quinoléine-3 carboxylate d'éthyle en solution dans 100 ml de toluène. Le mélange est agité une nuit à température ambiante, puis on verse dans 250 ml d'eau glacée. La solution aqueuse est lavée à l'éther, acidifiée par HCl dilué jusqu'à pH 7,1-7,2. Le précipité jaune clair obtenu est recristallisé dans l'acétate d'éthyle ; F = 224°C (Rendement 40%).

### EXEMPLE 4

### 8-chloro 3-hydroxy 1H-pyrrolo[3.2-c]1,5-naphtyridine-2 carboxylate d'éthyle

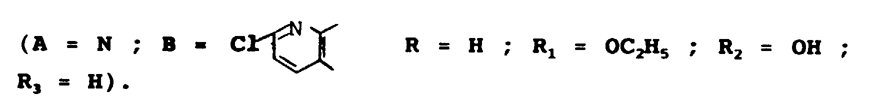

**a)[4-(3-éthoxycarbonyl-6-chloro)1,5-naphtyridyl]-2-aminoacétate d'éthyle.**
   A 5,1 g de 4,6-dichloro-3-éthoxycarbonyl 1,5-naphtyridine en solution dans 140 ml d'éthanol et 2,65 g du chlorhydrate de l'ester éthylique de la glycine est ajouté, en goutte à goutte, 5,2 ml de triéthylamine.
   L'addition terminée, le milieu réactionnel est porté deux heures à la température de reflux de l'éthanol, puis amené à sec ; le résidu est dissout dans le chlorure de méthylène et la phase organique est lavée à l'eau puis séchée. Après élimination du solvant, le produit est recristallisé dans l'éthanol. F = 140°C (Rendement 46%).
b) A 1,37 g de tert-butylate de potassium en solution dans 25 ml de tert-butanol est ajouté, en goutte à goutte, sous léger courant d'azote, 3,84 g de la naphtyridine précédente en solution dans 45 ml de toluène et 40 ml de diméthylformamide. La solution est agitée à température ambiante une nuit, puis le milieu réactionnel est versé dans 150 ml d'un mélange eau-glace. Après neutralisation de la phase aqueuse, le produit recherché précipite. F = 250°C (Rendement 59%).

### EXEMPLE 5

### 7-(N,N-dipropylcarbamoyl) 6-hydroxy 8-méthyl pyrrolo[3,2-d]thiéno[3, 2-b]pyridine

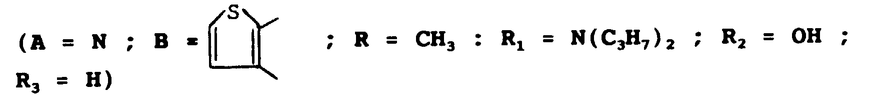

a) 7-(N(N,N-dipropylcarbamoylméthyl)N-méthylamino)thiéno[3,2-b]pyridine-6-carboxylate d'éthyle.
   3 g de 7-chloro thiéno[3,2-b]pyridine-6 carboxylate d'éthyle (F = 84°C), préparé par action de POCl₃ sur le 7-hydroxy thiéno[3,2-b]pyridine-6 carboxylate d'éthyle, obtenu par la méthode décrite dans J. Chem. Res. (M) p.4701 (1978), 2,6 g de N,N-dipropyl (2-méthylamino)acétamide et 2,3 ml de triéthylamine dans 70 ml de toluène sont portés à la température de reflux du milieu pendant 20 heures. La phase toluénique est lavée à l'eau puis concentrée à sec. Le produit attendu est purifié par chromatographie sur gel de silice en éluant à l'éther isopropylique ; c'est une huile jaune. (Rendement = 49%).
b) 2,3 g du produit précédent en solution dans 50 ml de toluène sec sont introduits dans 20 ml de tert-butanol contenant 0,8 g de tert-butylate de potassium. Après 3 heures à température ambiante, le mélange est versé dans 300 ml d'eau froide. La phase aqueuse séparée est filtrée puis acidifiée par addition d'une solution aqueuse de HCl 0,1N jusqu'à pH 7,2. Le précipité formé est filtré puis recristallisé dans l'acétate d'éthyle. F = 120°C (Rendement 40%).

### EXEMPLE 6

### 3-amino 8-(N,N-diéthylamino) 6-méthyl-1 H-pyrrolo[3,2-c]quinoléine-2 carboxylate d'éthyle.

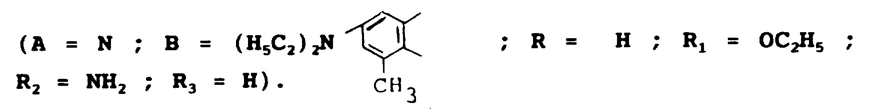

a) 3-[4-(N,N-diéthylamino) 2-méthyl anilino] 2-cyano acrylate d'éthyle
   Un mélange de 85 g de 4-(N,N-diéthylamino) 2-méthyl aniline et 67 g de 2-cyano 3-éthoxyacrylate d'éthyle est chauffé pendant 2 heures à 140°-150°C en distillant l'éthanol formé au fur et à mesure. Après recristallisation dans le cyclohexane, on obtient le produit cherché. F = 128°C (Rendement 93%).
b) 3-cyano 6-(N,N-diéthylamino) 4-hydroxy 8-méthyl quinoléine
   On introduit dans 645 ml d'oxyde de diphényle porté à 200°C, 60 g du produit précédent, puis on chauffe vers 257°C pendant 4 heures avant de distiller environ 400 ml d'oxyde de diphényle. Après refroidissement, le produit attendu est précipité par addition d'éther de pétrole ; le solide est lavé avec de l'éther éthylique puis de l'acétate d'éthyle chaud pour donner un solide brun. F = >250°C avec un rendement de 43%.
c) 4-chloro 3-cyano 6-(N,N-diéthylamino) 8-méthyl quinoléine
   Un mélange de 12 g du produit obtenu en b) et de 70 ml de chlorure de phosphoryle est chauffé pendant 1 h 30 à 70°C. Après évaporation sous vide du chlorure d'acide en excès, on verse dans l'eau, neutralise et extrait dans le chlorure de méthylène le produit chloré attendu. Il est purifié par filtration sur gel de silice dans un mélange cyclohexane-acétate d'éthyle (5-5) et recristallisé dans l'éther de pétrole. F = 115°C (Rendement 44%).
d) Un mélange de 5,8 g du produit obtenu en c), 4,2 g de phénol, 4,2 g de chlorhydrate de l'ester éthylique de la glycine et 8,6 ml de triéthylamine sont portés pendant 2 heures à 140°C.

On ajoute ensuite de l'eau dans le milieu, acidifie et lave avec de l'éther éthylique puis on neutralise la phase aqueuse avant d'extraire avec du chlorure de méthylène. Le résidu obtenu par évaporation du solvant est lavé avec de l'éther isopropylique puis recristallisé dans l'acétate d'éthyle. F = 256°C (rendement 51%).

### EXEMPLE 7

### 3-amino 8-(N,N-diéthylamino) -1,6-diméthyl pyrrolo[3,2-c]quinoléine 2-carboxylate d'éthyle.

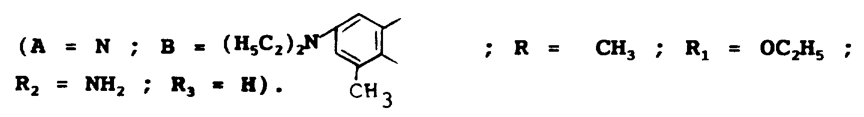

Un mélange de 3,4 g de 4-chloro 3-cyano 6-(N,N-diéthylamino) 8-méthyl quinoléine, de 2,6 g de phénol et de 2,8 g de chlorhydrate de l'ester éthylique de la sarcosine dans 5,1 ml de triéthylamine est porté pendant 2 heures à 140°C.

Après acidification par addition de HCl 0,1N et lavage à l'éther éthylique, la phase aqueuse est neutralisée puis extraite avec du chlorure de méthylène. Après séchage et évaporation du solvant organique, le résidu est chromatographié sur gel de silice en éluant avec de l'acétate d'éthyle. Le produit attendu est recristallisé dans l'éther isopropylique. F = 209°C (Rendement 36%).

### EXEMPLE 8

### 3-amino 8-(N,N-diéthylamino) 4-méthyl 1H-pyrrolo[3,2-c]quinoléine 2-carboxylate d'éthyle.

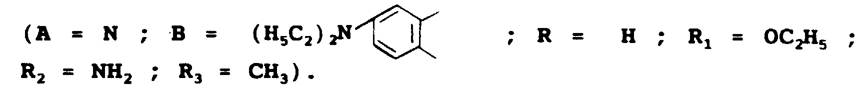

a) Un mélange équimoléculaire de 4-(N,N-diéthylamino)aniline et de 2-cyano 3-éthoxy 3-méthyl acrylate d'éthyle, préparé en appliquant la méthode décrite dans J. Org. Chem. 27, 1371-1374, (1961), est chauffé pendant 2 heures à 140°C.
   L'anilinoacrylate formé est alors cyclisé en solution dans l'oxyde de diphényle par chauffage à 260°C pendant 5 heures 30. Après traitement habituel, on obtient la 3-cyano 6-(N,N-diéthylamino) 4-hydroxy 2-méthyl quinoléine avec 49% de rendement. F >250°C.
b) 10 g de la quinoléine obtenue en a) sont chauffés à 70°C pendant 1 heure 30 dans 50 ml de chlorure de phosphoryle. Après évaporation à sec, addition d'eau et neutralisation, on extrait avec de l'acétate d'éthyle ; la phase organique est filtrée sur gel de silice, pour donner avec 53% de rendement le produit cherché. F = l14°C.
c) 5,8 g du dérivé chloré précédent avec 4,17 g de chlorhydrate de l'ester éthylique de la glycine, 4 g de phénol et 8,3 ml de triéthylamine sont portés pendant 2 heures à 140°C. Après acidification et lavage à l'éther éthylique, la phase aqueuse est neutralisée puis extraite avec du chlorure de méthylène. Le produit attendu recristallisé dans l'éther isopropylique fond à 250°C (rendement 37%).

### EXEMPLE 9

### 8-(N,N-diéthylamino)2-(N,N-dipropylcarba-moyl)3-éthoxy 1-méthyl pyrrolo[3,2-c]quinoléine (A = N ; B = 8-(C₂H₅)₂NC₆H₃ ; R = CH₃; R₁ = N(C₃H₇)₂ ; R₂ = OC₂H₅ ; R₃ = H)

On introduit, sous azote, dans 10 ml de diméthylformamide sec, contenant 0,24 g de NaH, une solution dans 30 ml du même solvant de 2 g de 8-(N,N-diéthylamino)2-(N,N-dipropylcarbamoyl)3-hydroxy 1-méthyl pyrrolo[3,2-c]quinoléine (F = 224°C), puis on chauffe l'ensemble à 60°C pendant 30 minutes avant d'ajouter goutte à goutte 0,4 ml d'iodure d'éthyle en solution dans 10 ml de solvant. Le mélange est chauffé pendant 2 heures à 70°C, puis versé dans 600 ml d'eau vers 10°C. Par extraction au chlorure de méthylène, on obtient 2,5 g de produit brut que l'on purifie par chromatographie sur gel de silice en éluant avec de l'acétate d'éthyle. L'huile obtenue est dissoute dans l'éther éthylique, et on ajoute une solution d'acide chlorhydrique dans le même solvant pour précipiter le chlorhydrate du produit recherché, avec 65% de rendement. F = 170°C.

### EXEMPLE 10

### 3-amino 8-(N,N-diéthylamino) 4-méthylthio 1H-pyrrolo[3,2-c]quinoléine 2-carboxylate d'éthyle.

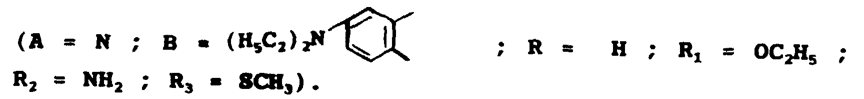

a) 3-[4-(N,N-diéthylamino)anilino] 2-cyano 3-méthylthio acrylate d'éthyle
   21,7 g de 2-cyano 3,3-bis-(méthylthio)acrylate d'éthyle et 16,5 g de 4-(N,N-diéthylamino)aniline sont chauffés pendant 2 heures à 120°C. Le résidu est purifié par chromatographie sur colonne de silice en éluant avec un mélange de toluène/éthanol (97/3) ; le produit attendu est recristallisé dans le cyclohexane. F = 100°C (Rendement 81%).
b) 6-(N,N-diéthylamino) 3-cyano 4-hydroxy 2-méthylthio quinoléine
   26 g du produit obtenu en a) sont cyclisés par chauffage dans du Dowthern A, mélange de 275 g de biphényle et 707 ml d'oxyde de diphényle, à la température de reflux du milieu pendant 45 minutes. Le solvant est ensuite éliminé par distillation sous pression réduite. Le résidu, après lavage par l'éther de pétrole, est purifié par chromatographie sur colonne de silice en éluant avec un mélange de toluène/éthanol (95/5).
c) 6-(N,N-diéthylamino) 4-chloro 3-cyano 2-méthylthio quinoléine
   100 ml de POCl₃ et 5 g de l'huile obtenue en b) sont chauffés à reflux pendant 1 heure. L'oxychlorure de phosphore en excès est éliminé, le résidu est dissous dans du dichlorométhane, et la phase organique est lavée avec une solution aqueuse de NaOH, puis à l'eau, jusqu'à neutralité ; après séchage, on concentre à sec, et le résidu est purifié par chromatographie sur colonne de silice (éluant toluène/éthanol 99/1).
   Après recristallisation dans le cyclohexane, le produit cherché fond à 146°C. (Rendement 72%).
d) [6-(N,N-diéthylamino) 3-cyano 2-méthylthio 4-quinolyl]amino acétate d'éthyle.
   2 g du composé obtenu en c), 1 g de chlorhydrate de l'ester éthylique de la glycine, 2 ml de triéthylamine et 100 ml d'éthanol sont chauffés à reflux dans l'éthanol pendant douze heures avant addition de la même quantité de chlorhydrate et de 2 ml de triéthylamine. Le reflux est à nouveau maintenu pendant 20 heures.
   Le milieu, après concentration à sec, est repris par le dichlorométhane,, la phase organique est lavée à l'eau. Après élimination du solvant, le résidu est chromatographié sur colonne de silice (éluant cyclohexane/acétate d'éthyle 80/20). On obtient avec 55% de rendement le produit attendu, qui fond à 130°C.
e) 1,5 g de ce composé sont cyclisés dans 60 ml d'éthanol contenant de l'éthylate de sodium, obtenu avec 0,1 g de Na, à température ambiante. Après 4 heures d'agitation, le milieu est concentré à sec, et on verse sur le résidu 100 ml d'eau. Le précipité est isolé. F = 198°C. (Rendement 30%).

Dans les tableaux 1 et 2 qui suivent sont mentionnés les points de fusion de dérivés du pyrrole selon l'invention préparés en appliquant l'une des méthodes précédentes.

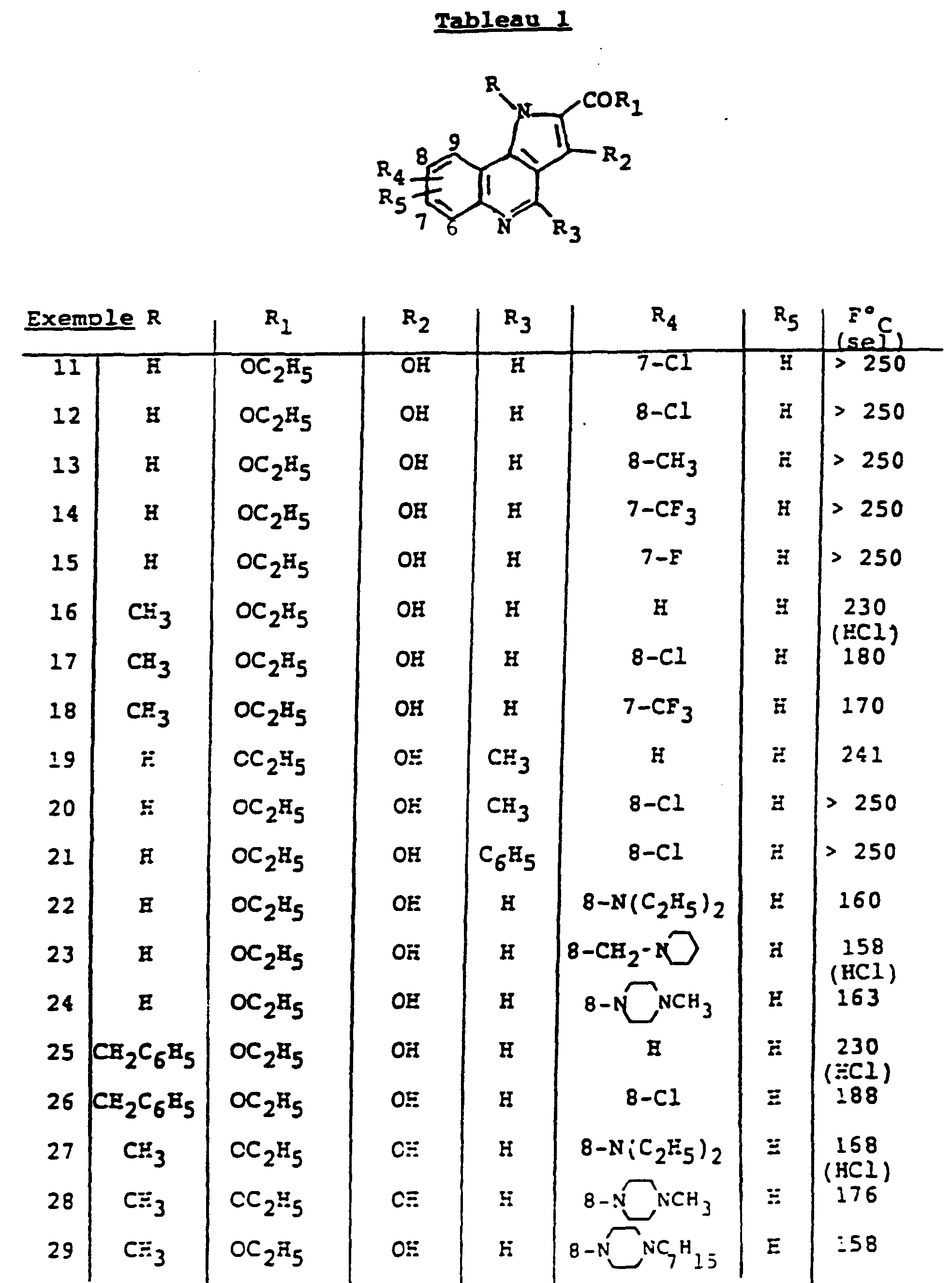

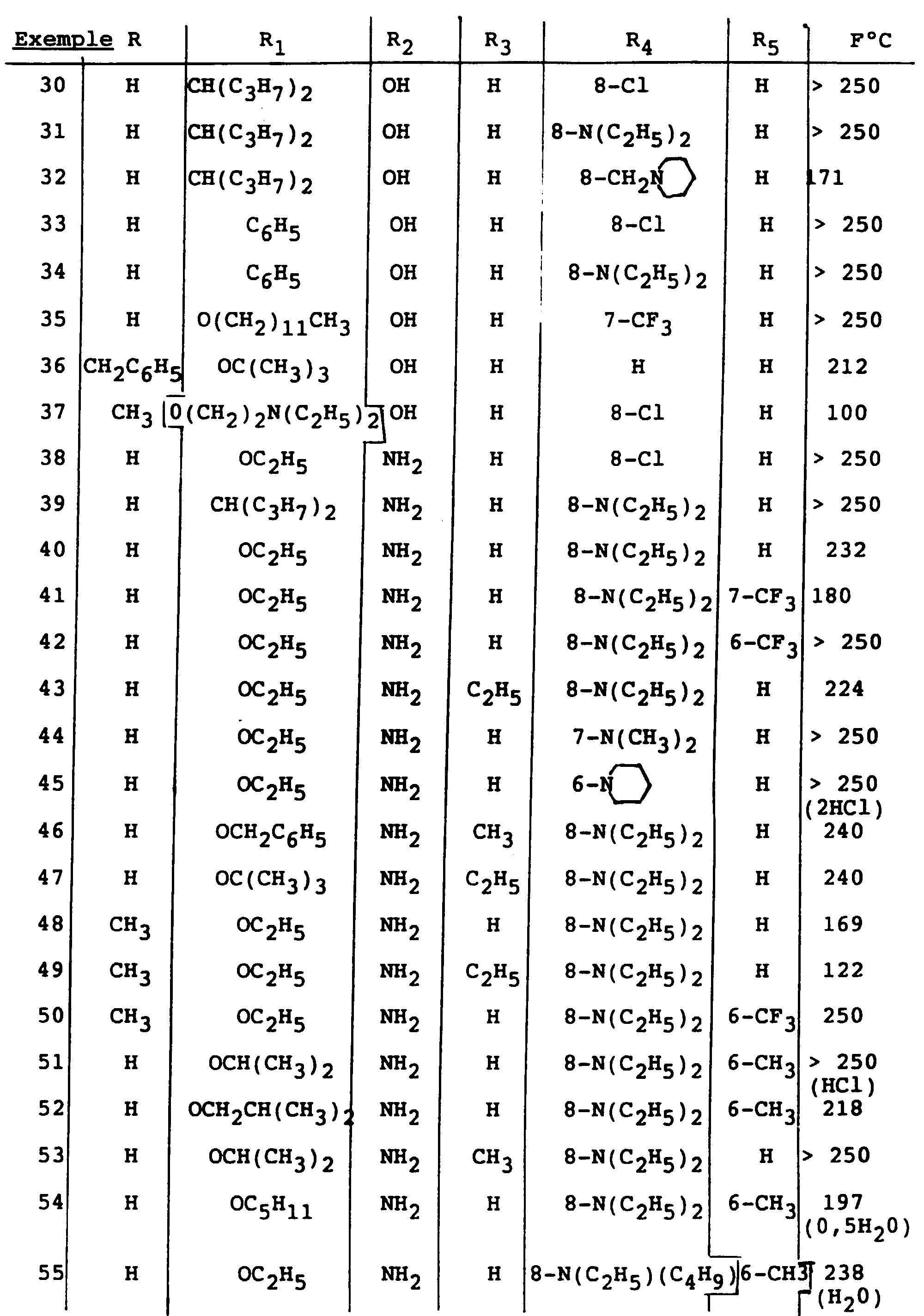

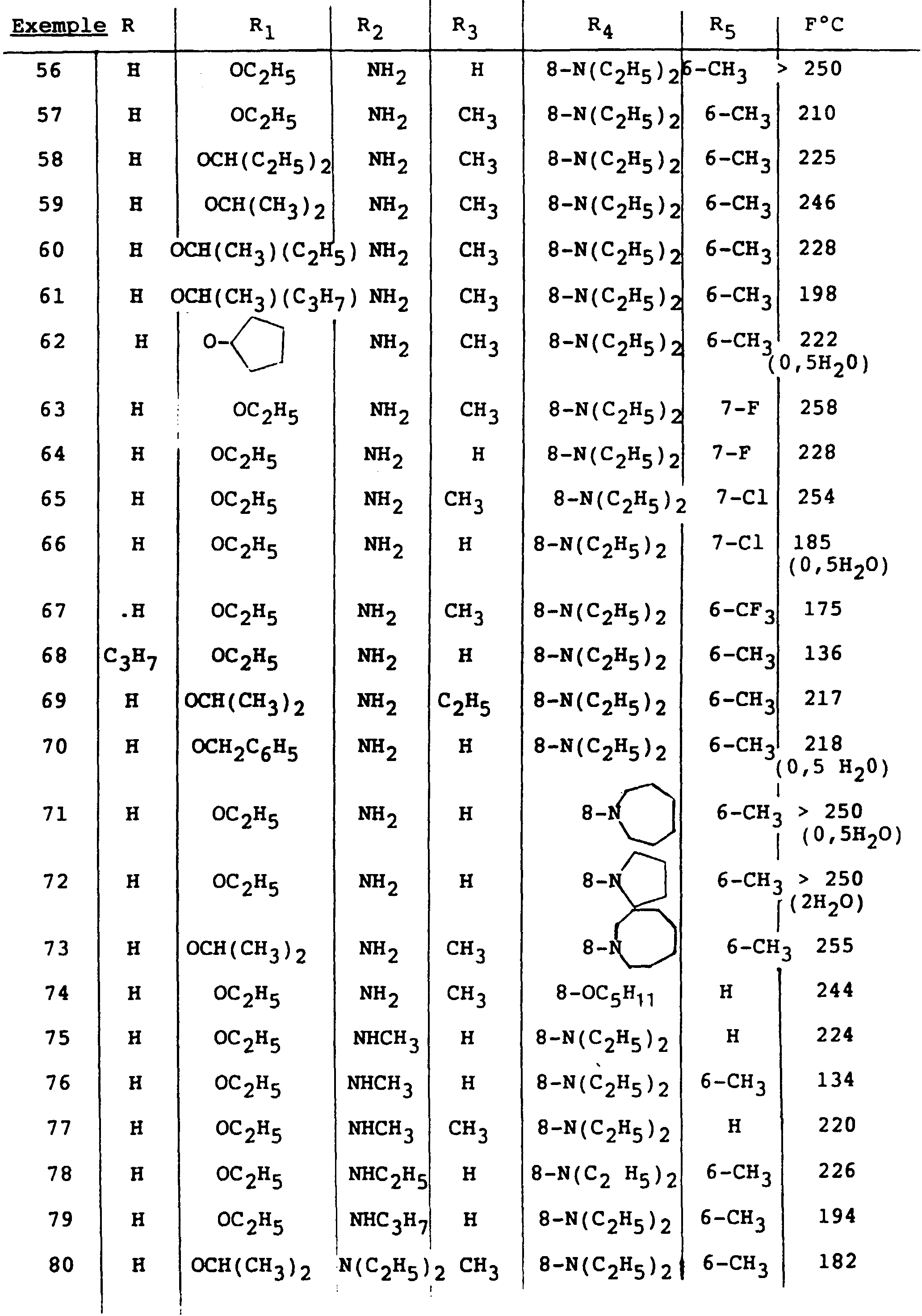

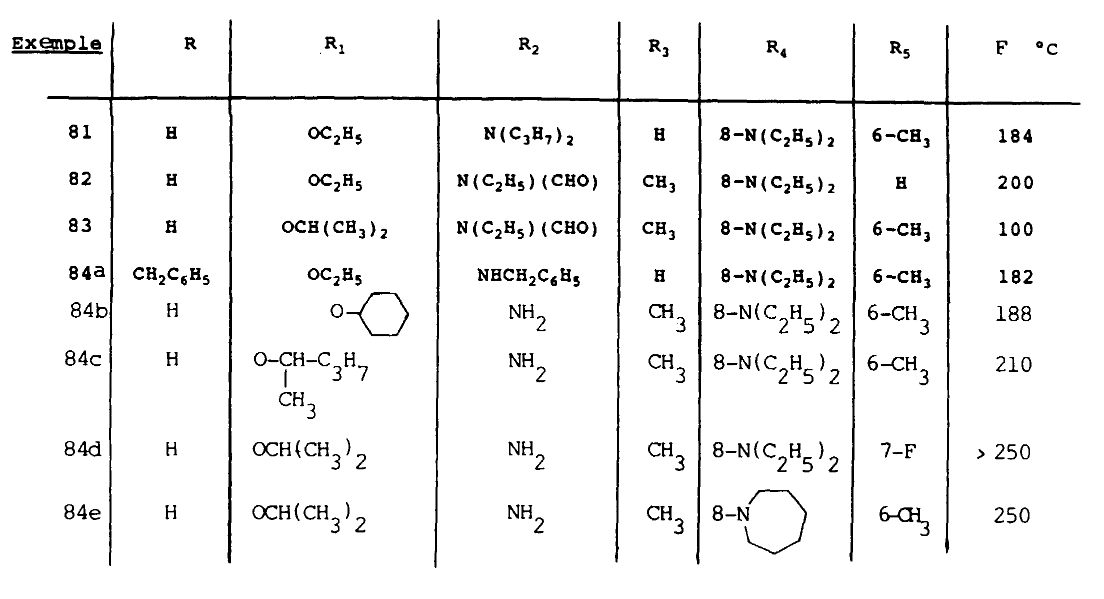

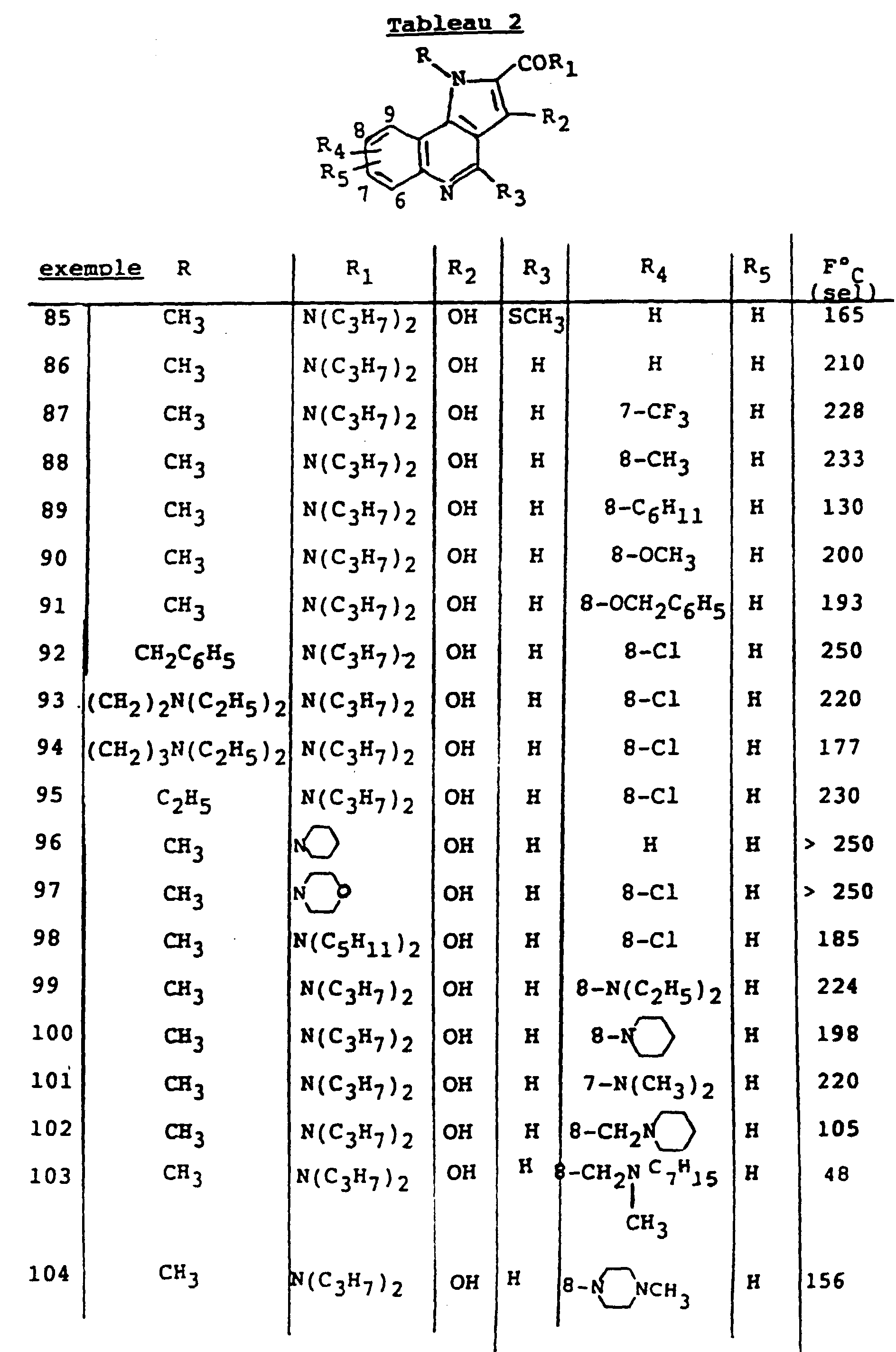

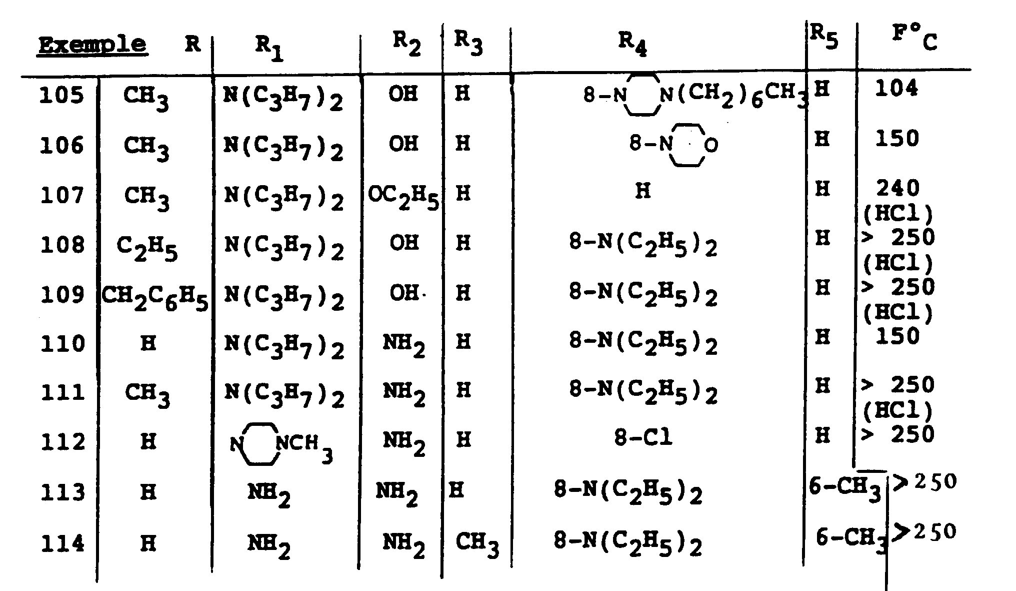

Dans le tableau 3 sont mentionnés des exemples de dérivés de naphtyridines.

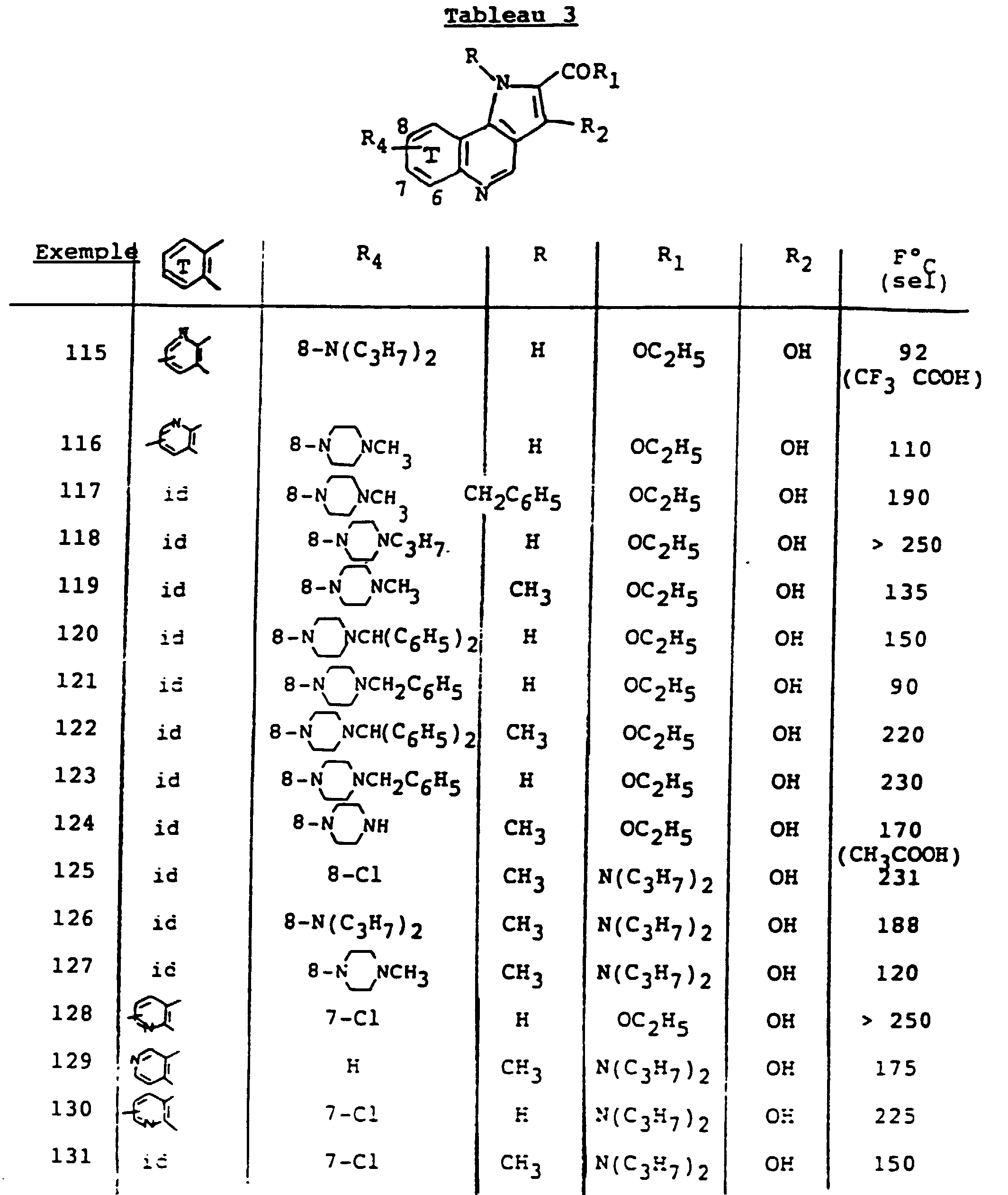

### EXEMPLE 132

### 3-amino 2-carbamoyl 8-(N,N-diéthylamino) 4-méthyl-1 H-pyrrolo[3, 2-c]quinoléine

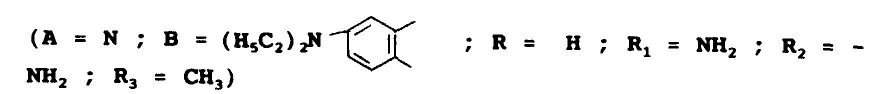

a) 4-(carbamoylméthylamino) 3-cyano 6-(N,N-diéthylamino) 2-méthyl quinoléine
   6,5 g de chloroquinoléine, préparée comme à l'exemple 8 b), 4,55 g de phénol, 3,7 g de chlorhydrate de glycinamide et 9,3 ml de triéthylamine sont chauffés pendant 2 heures à 140°C. Après refroidissement, le mé lange est acidifié par addition d'une solution aqueuse de HCl 0,1 N ; les cristaux obtenus sont lavés à l'éther éthylique et recristallisés dans l'éthanol. F = 225°C.
b) 1 g du produit précédent est ajouté peu à peu à 0,4 g de tert-butylate de potassium dans 50 ml de tert-butanol. Après 1 heure d'agitation, le mélange est versé dans l'eau froide, puis acidifié par addition d'une solution aqueuse de HCl dilué. Le chlorhydrate du produit attendu précipite après lavage avec de l'acétonitrile à chaud, il fond à plus de 260°C.

### EXEMPLE 133

### Acide 3-amino 8-(N,N-diéthylamino)4-éthyl-1 H-pyrrolo[3,2-c]quinoléine 2-carboxylique,

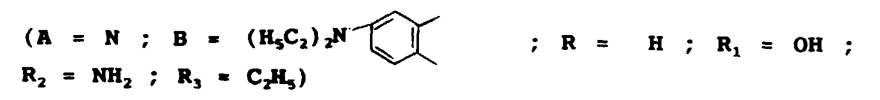

A une solution d'acétate d'éthyle saturée d'acide chlorhydrique gazeux, on ajoute peu à peu 0,5 g de 3-amino 8-(N,N-diéthylamino)4-éthyl 1-H pyrrolo[3,2-c]quinoléine 2-carboxylate de tert-butyle (F = 240°C). Le mélange est agité à température ambiante pendant 48 heures. Les cristaux obtenus sont filtrés, lavés à l'acétonitrile puis à l'isopropanol. F = 223°C.

### EXEMPLE 134

### 8-(N,N-diéthylamino)3-hydroxy thiéno [3,2-c]quinoléine 2-carboxylate d'éthyle

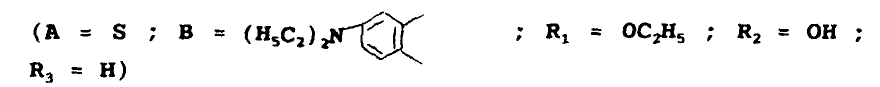

a) 6,1 g de 6-(N,N-diéthylamino) 4-chloroquinoléine 3-carboxylate d'éthyle, 2,6 g de thioglycolate d'éthyle et 3 ml de triéthylamine en solution dans 50 ml de diméthylformamide sont chauffés durant 3 heures à 70°C. Le solvant est évaporé, et on verse sur le résidu de l'eau et de l'éther éthylique ; après séparation de la phase éthérée, séchage, et évaporation du solvant, on chromatographie l'huile résiduelle sur gel de silice pour obtenir, avec 50% de rendement, le [4-(6-(N,N-diéthylamino)3-éthoxy carbonyl)quinolyl]2-thioacétate d'éthyle, huileux.
b) 1,6 g de ce produit en solution dans 50 ml de toluène sont introduits dans une solution de 0,5 g de tertiobutylate de potassium dans 8 ml de tertiobutanol. Le mélange est agité pendant 12 heures avant de concentrer à sec. Le résidu est repris à l'eau et la phase aqueuse est neutralisée par addition d'une solution aqueuse d'acide chlorhydrique diluée. Le précipité jaune formé est filtré puis recristallisé dans un mélange d'hexane et d'éther isopropylique (50/50) pour donner, avec 45% de rendement, le produit attendu qui fond à 142°C.

Dans le Tableau 4 figurent des exemples de dérivés de thiénoquinoléine et naphtyridine.

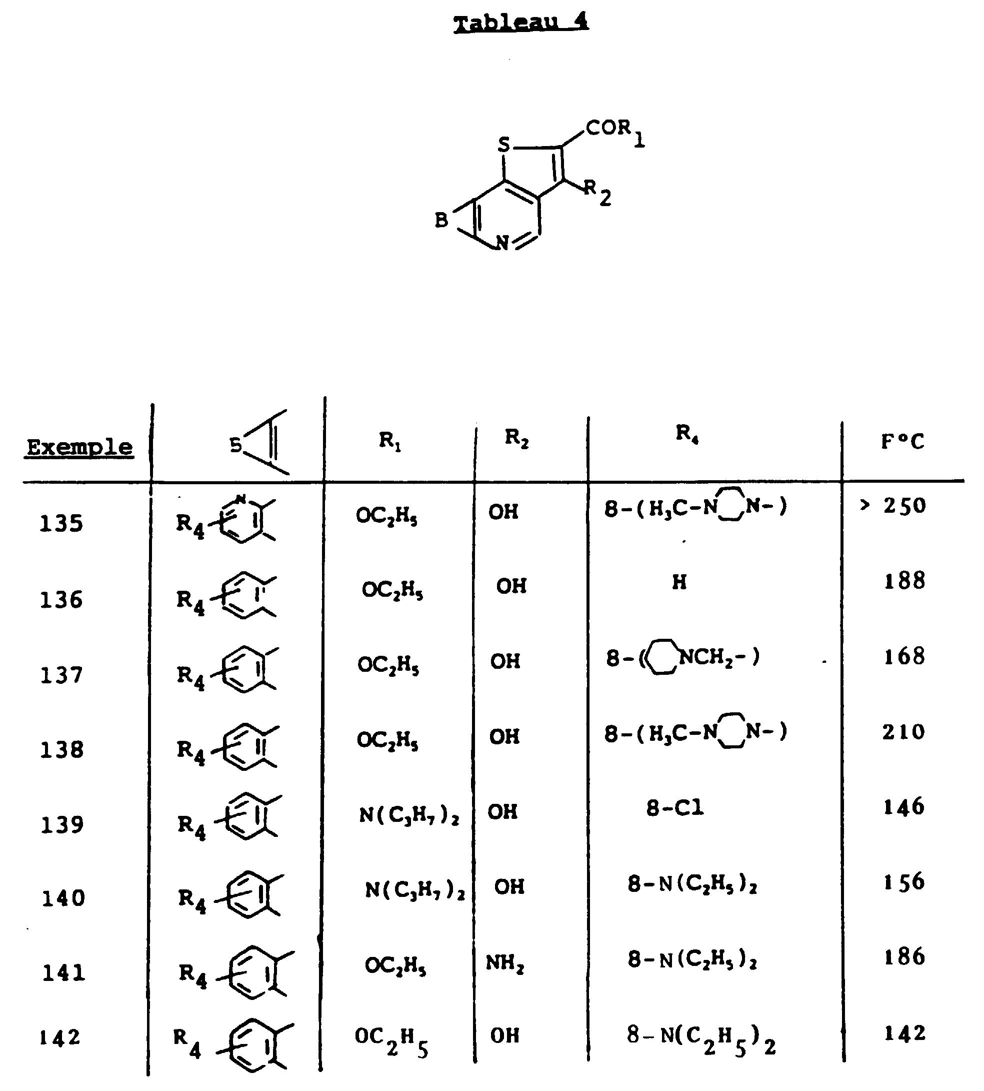

## Revendications

1. Composés de formule 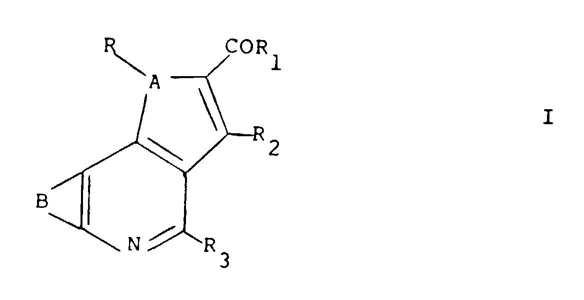 dans laquelle
R₁ représente OH, (C₁-C₁₂)alkyle, (C₁-C₁₂)alkoxy, benzyloxy, phényle, benzyle, (C₁-C₄)alkylNZ₁Z₂ ou NZ₁Z₂ ;
R₂ représente OH, SH, (C₁-C₄)alkoxy, (C₁-C₄)alkylthio ou NZ₁Z₂ ;
R₃ représente H, (C₁-C₄)alkyle, (C₁-C₄)alkylthio, (C₁-C₄)alkoxy, phényle ou benzyle ;
A représente S, et dans ce cas R ne représente rien, ou A représente N et R représente H ou (C₁-C₄)alkyle qui peut être substitué par phényle ou NZ₁Z₂ ;
B représente un noyau phényle, pyridyle ou thiényle qui est condensé avec le noyau pyridyle, et B est éventuellement substitué par un ou des groupes choisis parmi les halogéno, (C₁-C₆)alkyle, (C₁-C₆)alkoxy, CF₃, CH₂NZ₁Z₂ ou NZ₁Z₂ ; et
Z₁ et Z₂ représentent, indépendamment les uns des autres, H, (C₁-C₆)alkyle, formyle, benzyle, ou ils forment avec l'atome d'azote auquel ils sont attachés un hétérocycle saturé, éventuellement interrompu par un hétéroatome choisi parmi O, S ou N, ce dernier pouvant être substitué par (C₁-C₈)alkyle, benzyle, phényle ou diphénylméthyle, étant entendu que lorsque A est S, R ne représente rien, R₁ est -OCH₃, R₃ est H et B est thiényle, R₂ ne peut pas représenter -OH ou -OCH₃,
et leurs sels avec un acide ou une base.

2. Composés de formule I selon la revendication 1, dans laquelle
R₁ représente OH, (C₁-C₆)alkyle, (C₁-C₁₂)alkoxy, phényle ou NZ₁Z₂ ;
R₂ représente OH, SH, (C₁-C₄)alkoxy, (C₁-C₄)alkylthio ou NZ₁Z₂ ;
R₃ représente H, (C₁-C₄)alkyle, (C₁-C₄)alkylthio, (C₁-C₄)alkoxy, phényle ou benzyle ;
A représente S, et dans ce cas R ne représente rien, ou A représente N et R représente H ou (C₁-C₄)alkyle qui peut être substitué par phényle ou NZ₁Z₂ ;
B représente un noyau phényle, pyridyle ou thiényle qui est condensé avec le noyau pyridyle, et B est éventuellement substitué par un ou des groupes choisis parmi les halogéno, (C₁-C₆)alkyle, (C₁-C₄)alkoxy, CF₃, CH₂NZ₁Z₂ ou NZ₁Z₂ ; et
Z₁ et Z₂ représentent, indépendamment les uns des autres, H, (C₁-C₄)alkyle, benzyle, ou ils forment avec l'atome d'azote auquel ils sont attachés un hétérocycle saturé, éventuellement interrompu par un hétéroatome O, S ou N, ce dernier pouvant être substitué par (C₁-C₄)alkyle, benzyle ou phényle,
et leurs sels avec un acide ou une base.

3. Composés selon la revendication 1, de formule 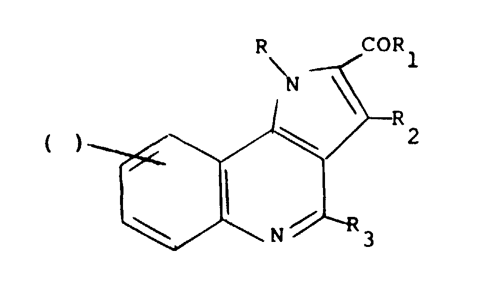 dans laquelle R, R₁, R₂, et R₃ ont la même signification qu'à la revendication 1, et le noyau phényle peut être substitué comme B à la revendication 1, et leurs sels.

4. Composés selon la revendication 1, de formule 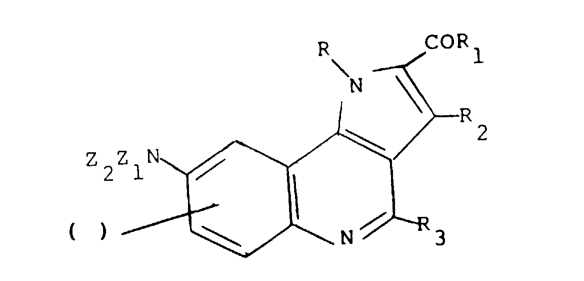 dans laquelle le noyau phényle peut être substitué par halogéno, CF₃, (C₁-C₆)alkyle ou (C₁-C₆)alkoxy, et Z₁ et Z₂ représentent, indépendamment l'un de l'autre, H, (C₁-C₆)alkyle, formyle, benzyle, ou forment avec l'atome d'azote auquel ils sont attachés un hétérocycle saturé qui peut contenir un hétéroatome choisi parmi 0, S ou N, ce dernier pouvant être substitué par (C₁-C₄)alkyle, benzyle ou phényle, et leurs sels.

5. Composés selon la revendication 4, dans lesquels R₂ représente NZ'₁Z'₂, où Z'₁ et Z'₂ représentent indépendamment l'un de l'autre H ou (C₁-C₆)alkyle et COR₁ est un groupe ester.

6. Composés selon la revendication 1, de formule 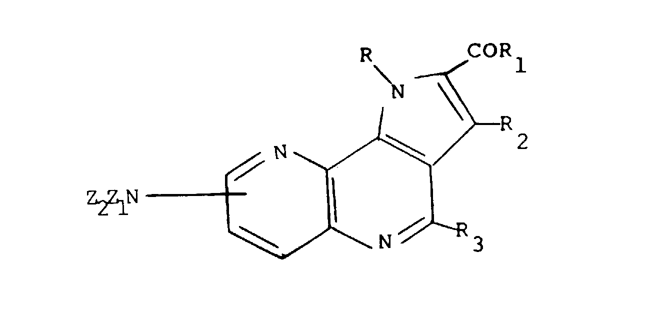 dans laquelle R, R₁, R₂, et R₃ ont la même signification qu'à la revendication 1, et Z₁ et Z₂ représentent H, (C₁-C₆)alkyle ou forment avec l'atome d'azote auquel ils sont attachés un groupe piperazino pouvant être substitué sur l'azote par (C₁-C₈)alkyle, benzyle, phényle, ou diphénylméthyle, et leurs sels.

7. Procédé de préparation des composés de formule I selon l'une des revendications 1 à 6, caractérisé en ce que l'on cyclise, dans une réaction de Dieckmann, le composé de formule 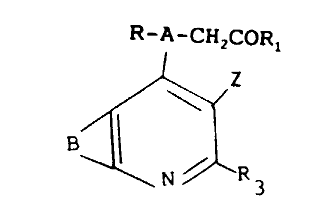 dans laquelle A, B, R, R₁ et R₃ ont la même signification qu'à la revendication 1, et Z représente soit un groupe alkoxycarbonyle, pour former un composé de formule I dans laquelle R₂ représente OH, soit un groupe cyano pour former un composé de formule I dans laquelle R₂ représente NH₂, le composé pouvant ensuite être substitué pour obtenir un composé de formule I dans laquelle R₂ est (C₁-C₄)alkoxy, (C₁-C₄)alkylthio ou NZ₁Z₂, où R₂, Z₁ et Z₂ sont tels que définis à la revendication 1.

8. Procédé de préparation d'un composé de formule I selon l'une des revendications 1 à 6, caractérisé en ce que l'on chauffe, en présence de phénol, les composés de formules 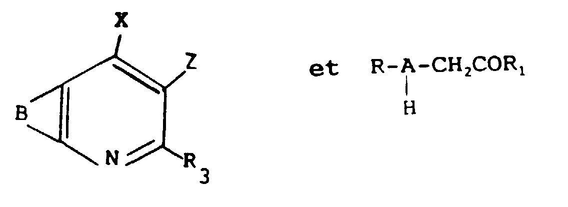 dans lesquelles A, B, R, R₁ et R₃ ont la même signification que dans la revendication 1, X représente Cl et Z représente soit un groupe alkoxycarbonyle, pour former un composé de formule I dans laquelle R₂ représente OH, soit un groupe cyano pour former un composé de formule I dans laquelle R₂ représente NH₂ ledit composé pouvant être substitué pour obtenir un composé de formule I dans laquelle R₂ est (C₁-C₄)alkoxy, (C₁-C₄)alkylthio ou NZ₁Z₂, où R₂, Z₁ et Z₂ sont tels que définis à la revendication 1.

9. Composition pharmaceutique caractérisée en ce qu'elle contient une quantité thérapeutiquement efficace d'un composé selon l'une des revendications 1 à 6, éventuellement salifié avec un acide ou une base pharmaceutiquement acceptable.

## Patentansprüche

1. Verbindungen der Formel 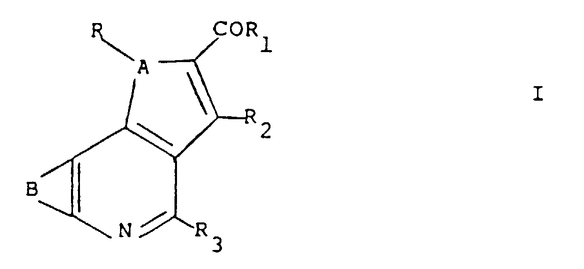 in der
R₁ OH, (C₁-C₁₂)-Alkyl, (C₁-C₁₂)-Alkoxy, Benzyloxy, Phenyl, Benzyl, (C₁-C₄)-Alkyl-NZ₁Z₂ oder NZ₁Z₂;
R₂ OH, SH, (C₁-C₄)-Alkoxy, (C₁-C₄)-Alkylthio oder NZ₁Z₂;
R₃ H, (C₁-C₄)-Alkyl, (C₁-C₄)-Alkylthio, (C₁-C₃)-Alkoxy, Phenyl oder Benzyl; A S und in diesem Fall R nichts oder
AN und R H oder (C₁-C₄)-Alkyl, welches durch Phenyl oder NZ₁Z₂ substituiert sein kann;
B einen Phenyl-, Pyridyl- oder Thienylkern, der mit dem Pyridylkern kondensiert ist und B gegebenenfalls durch eine oder mehrere Gruppen ausgewählt aus Halogen, (C₁-C₆)-Alkyl, (C₁-C₆)-Alkoxy, CF₃, CH₂NZ₁Z₂ oder NZ₁Z₂ substituiert ist; und
Z₁ und Z₂ unabhängig voneinander H, (C₁-C₆)-Alkyl, Formyl, Benzyl oder gemeinsam mit dem Stickstoffatom, an das sie gebunden sind, einen gesättigten Heterocyclus, der gegebenenfalls durch ein Heteroatom ausgewählt aus O, S oder N unterbrochen ist, wobei dieses letztere durch (C₁-C₆)-Alkyl, Benzyl, Phenyl oder Diphenylmethyl substituiert sein kann, bedeuten, mit der Maßgabe, daß, wenn A S bedeutet, R nichts, R₁ -OCH₃, R₃ H und B Thienyl darstellen und R₂ nicht -OH oder -OCH₃ darstellt,
und deren Salze mit einer Säure oder Base.

2. Verbindungen der Formel I nach Anspruch 1, in der
R₁ OH, (C₁-C₆)-Alkyl, (C₁-C₁₂)-Alkoxy, Phenyl oder NZ₁Z₂;
R₂ OH, SH, (C₁-C₄)-Alkoxy, (C₁-C₄)-Alkylthio oder NZ₁Z₂;
R₃ H, (C₁-C₄)-Alkyl, (C₁-C₄)-Alkylthio, (C₁-C₄)-Alkoxy, Phenyl oder Benzyl;
A S und in diesem Fall R nichts oder
A N und R H oder (C₁-C₄)-Alkyl, welches durch Phenyl oder NZ₁Z₂ substituiert sein kann;
B einen Phenyl-, Pyridyl- oder Thienylkern, der mit dem Pyridylkern kondensiert ist und B gegebenenfalls durch eine oder mehrere Gruppen ausgewählt aus Halogen, (C₁-C₆)-Alkyl, (C₁-C₄)-Alkoxy, CF₃, CH₂NZ₁Z₂ oder NZ₁Z₂ substituiert ist; und
Z₁ und Z₂ unabhängig voneinander H, (C₁-C₄)-Alkyl, Benzyl oder gemeinsam mit dem Stickstoffatom, an das sie gebunden sind, einen gesättigten Heterocyclus, der gegebenenfalls durch ein Heteroatom O, S oder N unterbrochen ist, wobei dieses letztere durch C₁-C₄)-Alkyl, Benzyl oder Phenyl substituiert sein kann, bedeuten,
und deren Salze mit einer Säure oder Base.

3. Verbindungen nach Anspruch 1 der Formel 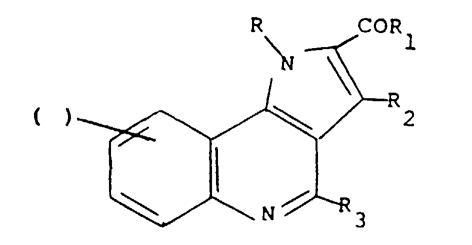 in der R, R₁, R₂ und R₃ die in Anspruch 1 angegebenen Bedeutungen besitzen und der Phenylkern wie B in Anspruch 1 angegeben substituiert sein kann, und deren Salze.

4. Verbindungen nach Anspruch 1 der Formel 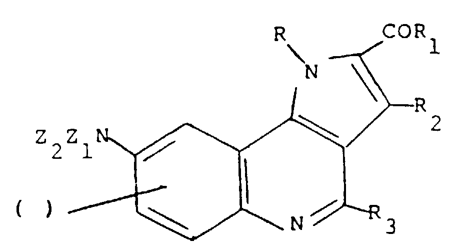 in welcher der Phenylkern durch Halogen, CF₃, (C₁-C₆)-Alkyl oder (C₁-C₆)-Alkoxy substituiert sein kann und Z₁ und Z₂ unabhängig voneinander H, (C₁-C₆)-Alkyl, Formyl, Benzyl oder gemeinsam mit dem Stickstoffatom, an das sie gebunden sind, einen gesättigten Heterocyclus, der ein Heteroatom ausgewählt aus O, S oder N enthalten kann, wobei dieses letztere durch (C₁-C₄)-Alkyl, Benzyl oder Phenyl substituiert sein kann, bedeuten, und deren Salze.

5. Verbindungen nach Anspruch 4, worin R₂ NZ'₁Z'₂ bedeutet, worin Z'₁ und Z'₂ unabhängig voneinander H oder (C₁-C₆)-Alkyl bedeuten, und COR₁ eine Estergruppe darstellt.

6. Verbindungen nach Anspruch 1 der Formel 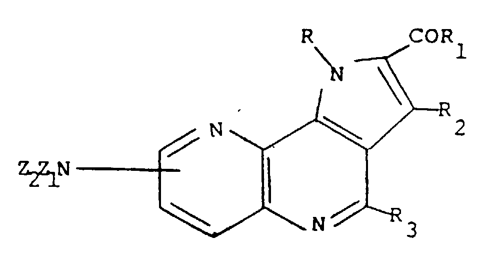 in der R, R₁, R₃ und R₃ die in Anspruch 1 angegebenen Bedeutungen besitzen und Z₁ und Z₂ H, (C₁-C₆)-Alkyl oder zusammen mit dem Stickstoffatom, an das sie gebunden sind, eine Piperazinogruppe bedueten, welche am Stickstoff durch (C₁-C₈)-Alkyl, Benzyl, Phenyl oder Diphenylmethyl substituiert sein kann, und deren Salze.

7. Verfahren zur Herstellung der Verbindungen der Formel I nach einem der Ansprüche 1 bis 6, dadurch gekennzeichnet, daß man im Rahmen einer Dieckmann-Reaktion die Verbindung der Formel cyclisiert: 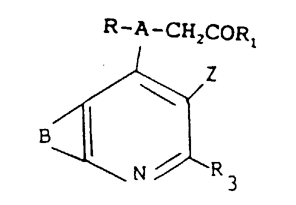 in der A, B, R, R₁ und R₃ die in Anspruch 1 angegebenen Bedeutungen besitzen, und Z entweder eine Alkoxycarbonylgruppe darstellt, zur Bildung einer Verbindung der Formel I, in der R₂ OH darstellt, oder eine Cyanogruppe darstellt zur Bildung einer Verbindung der Formel I, in der R₂ NH₂ darstellt, welche Verbindung anschließend substituiert werden kann zur Bildung einer Verbindung der Formel I, in der R₂ (C₁-C₄)-Alkoxy, (C₁-C₄)-Alkylthio oder NZ₁Z₂ darstellt, wobei R₂, Z₁ und Z₂ die in Anspruch 1 angegebenen Bedeutungen besitzen.

8. Verfahren zur Herstellung einer Verbindung der Formel I nach einem der Ansprüche 1 bis 6, dadurch gekennzeichnet, daß man die Verbindungen der Formeln in Gegenwart von Phenol erhitzt: 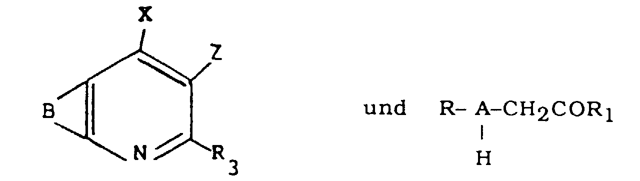 worin A, B, R, R₁ und R₃ die in Anspruch 1 angegebenen Bedeutungen besitzen, X Cl darstellt und Z entweder eine Alkoxycarbonylgruppe bedeutet, zur Bildung einer Verbindung der Formel I, in der R₂ OH darstellt, oder eine Cyanogruppe bedeutet, zur Bildung einer Verbindung der Formel I, in der R₂ NH₂ darstellt, welche Verbindung substituiert werden kann zur Bildung einer Verbindung der Formel I, in der R₂ (C₁-C₄)-Alkoxy, (C₁-C₄)-Alkylthio oder NZ₁Z₂ bedeutet, worin R₂, Z₁ und Z₂ die in Anspruch 1 angegebenen Bedeutungen besitzen.

9. Pharmazeutische Zubereitung, dadurch gekennzeichnet, daß sie eine therapeutisch wirksame Menge einer Verbindung nach einem der Ansprüche 1 bis 6 gegebenenfalls in Form eines Salzes mit einer pharmazeutisch annehmbaren Säure oder Base enthält.

## Claims

1. Compounds of formula 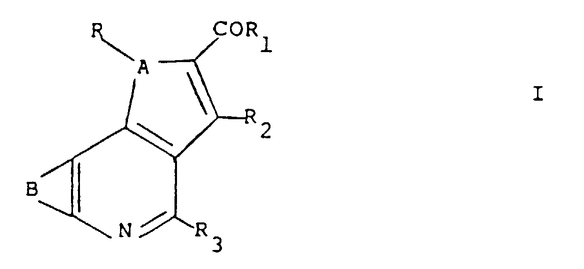 wherein
R₁ represents OH, (C₁₋₁₂)alkyl, (C₁₋₁₂)alkoxy, benzyloxy, phenyl, benzyl, (C₁₋₄)alkylNZ₁Z₂ or NZ₁Z₂ ;
R₂ represents OH, SH, (C₁₋₄)alkoxy, (C₁₋₄)alkylthio or NZ₁Z₂ ;
R₃ represents H, (C₁₋₄)alkyl, (C₁₋₄)alkylthio, (C₁₋₄)alkoxy, phenyl or benzyl ;
A represents S, and in this case R represents nothing, or
A represents N and R represents H or (C₁₋₄)alkyl which may be substituted by phenyl or NZ₁Z₂ ;
B represents a phenyl, pyridyl or thienyl nucleus which is condensed with the pyridyl nucleus, and B is optionally substituted by one or more groups selected from the halo, (C₁₋₆)alkyl, (C₁₋₆)alkoxy, CF₃, CH₂NZ₁Z₂ or NZ₁Z₂ groups; and
Z₁ and Z₂, independently of one another, represent H, (C₁₋₆)alkyl, formyl, benzyl, or together with the nitrogen atom to which they are attached they form a saturated heterocyclic group optionally interrupted by a heteroatom selected from O, S and N, whilst the latter may be substituted by (C₁₋₈)alkyl, benzyl, phenyl or diphenylmethyl, given that, when A is S, R represents nothing, R₁ is -OCH₃, R₃ is H and B is thienyl, R₂ cannot represent -OH or -OCH_{3,}
and the salts thereof with an acid or base.

2. Compounds of formula I according to claim 1, wherein
R₁ represents OH, (C₁₋₆)alkyl, (C₁₋₁₂)alkoxy, phenyl or NZ₁Z₂;
R₂ represents OH, SH, (C₁₋₄)alkoxy, (C₁₋₄)alkylthio or NZ₁Z₂;
R₃ represents H, (C₁₋₄)alkyl, (C₁₋₄)alkylthio, (C₁₋₄)alkoxy, phenyl or benzyl ;
A represents S, and in this case R represents nothing, or
A represents N and R represents H or (C₁₋₄)alkyl which may be substituted by phenyl or NZ₁Z₂ ;
B represents a phenyl, pyridyl or thienyl nucleus which is condensed with the pyridyl nucleus, and B is optionally substituted by one or more groups selected from the halo, (C₁₋₆)alkyl, (C₁₋₄)alkoxy, CF₃, CH₂NZ₁Z₂ or NZ₁Z₂ groups; and
Z₁ and Z₂, independently of one another, represent H, (C₁₋₆)alkyl, benzyl, or together with the nitrogen atom to which they are attached they form a saturated heterocyclic group optionally interrupted by an O, S or N heteroatom, whilst the latter may be substituted by (C₁₋₄)alkyl, benzyl or phenyl,
and the salts thereof with an acid or base.

3. Compounds according to claim 1, of the formula 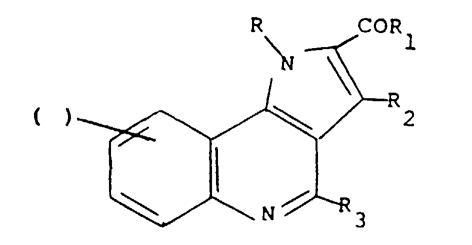 wherein R, R₁, R₂ and R₃ are defined as in claim 1, and the phenyl nucleus may be substituted like B in claim 1, and the salts thereof.

4. Compounds according to claim 1, of the formula 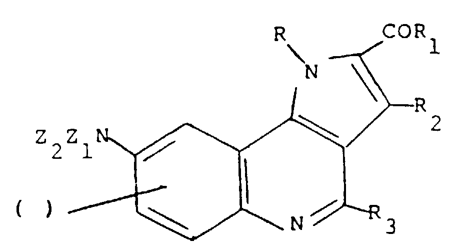 wherein the phenyl nucleus may be substituted by halo, CF₃, (C₁₋₆)alkyl or (C₁₋₆)alkoxy, and Z₁ and Z₂, independently of one another, represent H, (C₁₋₆)alkyl, formyl, benzyl, or together with the nitrogen atom to which they are attached they form a saturated heterocyclic group which may contain a heteroatom selected from O, S and N, whilst the latter may be substituted by (C₁₋₄)alkyl, benzyl or phenyl, and the salts thereof.

5. Compounds according to claim 4, wherein R₂ represents NZ'₁Z'₂, where Z'₁ and Z'₂ independently of each other denote H or (C₁₋₆)alkyl and COR₁ is an ester group.

6. Compounds according to claim 1, of the formula 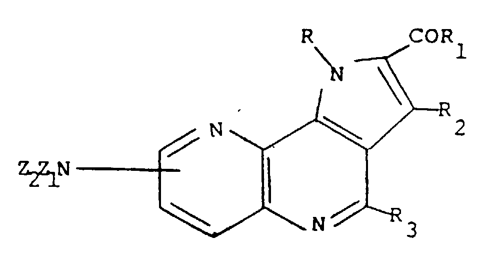 wherein R, R₁, R₂ and R₃ are defined as in claim 1, and Z₁ and Z₂ represent H, (C₁₋₆)alkyl or, together with the nitrogen atom to which they are attached, form a piperazino group which may be substituted at the nitrogen by (C₁₋₈)alkyl, benzyl, phenyl or diphenylmethyl, and the salts thereof.

7. Process for preparing compounds of formula I according to one of claims 1 to 6, characterised in that the compound of formula 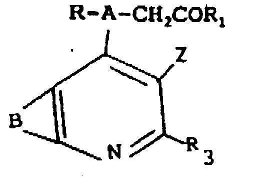 wherein A, B, R, R₁ and R₃ have the same meanings as in claim 1 and Z represents either an alkoxycarbonyl group, to form a compound of formula I wherein R₂ represents OH, or a cyano group to form a compound of formula I wherein R₂ represents NH₂, is cyclised in a Dieckmann reaction, after which the compound may be substituted to obtain a compound of formula I wherein R₂ is (C₁₋₄)alkoxy, (C₁₋₄)alkylthio or NZ₁Z₂, wherein R₂, Z₁ and Z₂ are as defined in claim 1.

8. Process for preparing a compound of formula I according to one of claims 1 to 6, characterised in that the compounds of formulae 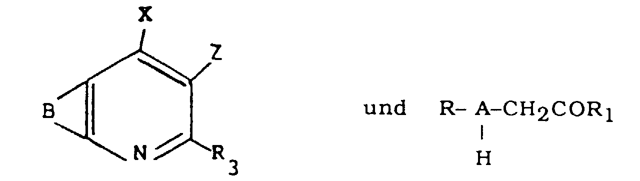 wherein A, B, R, R₁ and R₃ have the same meanings as in claim 1, X denotes Cl and Z represents either an alkoxycarbonyl group, to form a compound of formula I wherein R₂ represents OH, or a cyano group to form a compound of formula I wherein R₂ represents NH₂, whilst the said compound may be substituted to obtain a compound of formula I wherein R₂ is (C₁₋₄)alkoxy, (C₁₋₄)alkylthio or NZ₁Z₂, wherein R₂, Z₁ and Z₂ are as defined in claim 1, are heated in the presence of phenol.

9. Pharmaceutical composition, characterised in that it contains a therapeutically effective quantity of a compound according to one of claims 1 to 6, optionally converted into a salt with a pharmaceutically acceptable acid or base.
